(19) Europäisches Patentamt
European Patent Office
Office européen des brevets

(11) Veröffentlichungsnummer : **0 097 926 B2**

(12) # NEUE EUROPÄISCHE PATENTSCHRIFT

(45) Veröffentlichungstag der neuen Patentschrift : **21.08.91 Patentblatt 91/34**

(21) Anmeldenummer : **83106177.5**

(22) Anmeldetag : **24.06.83**

(51) Int. Cl.[5] : **C07C 211/63**, C07D 213/20, C07C 65/01, C07C 309/03, C07C 63/70, C07C 205/53, C07C 51/41, F17D 1/16

(54) **Quartäre Ammoniumsalze und deren Verwendung als Strömungsbeschleuniger.**

(30) Priorität : **29.06.82 DE 3224148**

(43) Veröffentlichungstag der Anmeldung : **11.01.84 Patentblatt 84/02**

(45) Bekanntmachung des Hinweises auf die Patenterteilung : **29.04.87 Patentblatt 87/18**

(45) Bekanntmachung des Hinweises auf die Entscheidung über den Einspruch : **21.08.91 Patentblatt 91/34**

(84) Benannte Vertragsstaaten : **BE CH DE FR GB LI NL SE**

(56) Entgegenhaltungen :
**EP-A- 0 091 086**
**WO-A-83/01583**
**FR-A- 1 518 427**
**US-A- 4 016 984**
**CHEMICAL ABSTRACTS, Band 56, No. 10, 14. Mai 1962, columbus, Ohio, USA,KIYOSHA FUTAKI "Effects of some addition agents on the rate of direct and physical development of liquid photographic emulsion",linke Spalte, Zusammenfassung No. 11 109i**
**CHEMICAL ABSTRACTS, Band 95, No. 19, 9. November 1981, Columbus, Ohio, USA,M. MASSACCESI "Two-phase titration of some quaternary ammonium compounds in the presence of amines or other quaternary ammonium compounds",Seite 437, linke Spalte, Zusammenfassung No. 175 879g**
**CHEMICAL ABSTRACTS, Band 58, No. 11, 27. Mai 1963, Columbus, Ohio, USA,J.A. SPRUNG "Photographic light-sensitive silver halide-polyvinyl alcohol emulsions",rechte Spalte, Zusammenfassung No. 11 376d**
**CHEMICAL ABSTRACTS, Band 95, No. 4, 27. Juli 1981, Columbus, Ohio, USA,T.V. STUPNIKOVA et al. "N-Cetylpicolinium bromides as additives which reduce the hydrodynamic resistance of water",Seite 114, Zusammenfassung No. 27 174h**
**NATURE, Band 214, 1967, London,A. WHITE "Flow characteristics of complex soap systems",Seiten 585, 586**

(56) Entgegenhaltungen :
**CHEMICAL ABSTRACTS, Band 91, No. 4, 23. Juli 1979, Columbus, Ohio, USA,S. GRAVSHOLT "Physicochemical properties of highly dilute aqueous detergent solutions showing viscoelastic behaviour (Viscosity measurement)",Seite 436, Zusammenfassung No. 27 461s**
**BERICHTE DER BUNDESGESELLSCHAFT FüR PHYSIKALISCHE CHEMIE, Band 85, April 1981, Weinheim,H. HOFFMANN et al. "Viskoelastische Tensidlösungen",Seiten 255-266**
**JOURNAL OF PHARMACEUTICAL SCIENCES, vol. 55, N:o 12 (Dez. 1966), 1395 - 1399**
**JOURNAL OF PHARMACEUTICAL SCIENCES, vol. 56, N.o 6 (Juni 1967), 743 - 747**
**JOURNAL OF PHARMACEUTICAL SCIENCES, vol. 57, no 11 (Nov. 1968), 1903 - 1906**
**E. SCHRAMM: "Einführung in die praktische Viskosimetrie (Firmenschrift Haake) 1981, Seiten 12 - 16**
**RHEOMETRY: Industrial Applications (Prof. Kenneth Walters) ResearchStudies Press (1980), Seiten 61 - 63**
**THE JOURNAL OF PHYSICAL CHEMISTRY, vol. 83, No 17 (1979), 2232 - 2236**

(73) Patentinhaber : **HOECHST AKTIENGESELLSCHAFT**
**Postfach 80 03 20**
**W-6230 Frankfurt am Main 80 (DE)**

(72) Erfinder : **Ohlendorf, Dieter, Dr.**
**Am Kühlen Grund 4**
**W-6237 Liederbach (DE)**
Erfinder : **Hoffmann, Heinz, Prof. Dr.**
**Waldsteinring 40**
**W-8580 Bayreuth (DE)**
Erfinder : **Interthal, Werner, Dr.**
**Dr. Ludwig-Opel-Strasse 62**
**W-6090 Rüsselsheim (DE)**
Erfinder : **Pintschovius, Ulrich, Dr.**
**Bad Sodener Strasse 6**
**W-6234 Hattersheim am Main (DE)**

EP 0 097 926 B2

Jouve, 18, rue Saint-Denis, 75001 PARIS

## Beschreibung

Es ist allgemein bekannt, daß turbulent strömende Flüssigkeiten an den sie begrenzenden Wänden einen Reibungswiderstand erfahren. Es ist auch bekannt, daß man diesen Reibungswiderstand durch Zusatz geringer Mengen bestimmter Stoffe herabsetzen kann. Stoffe, die diese Wirkung zeigen, werden im englischen Sprachgebrauch als "drag reducing agents" bezeichnet. Im deutschsprachigen Baum wird für diese Stoffe die Bezeichnung "Strömungsbeschleuniger" verwendet (im folgenden abgekürzt durch SB). Unter einem Strömungsbeschleuniger versteht man danach einen Stoff, der in geringer Menge einer turbulent oder pulsierend strömenden Flüssigkeit zugesetzt, diese Flüssigkeit - unter sonst gleichen Bedingungen - schneller strömen läßt. Strömungsbeschleuniger bewirken, daß man mit einer gegebenen Pumpe durch eine gegebene Rohrleitung mehr Flüssigkeit fördern kann.

In vielen Fällen ist allein schon diese Tatsache ein technischer Gewinn, wenn z. B. eine Rohrleitung im Normalbetrieb voll ausgelastet ist und zu bestimmten Zeiten ein Spitzenverbrauch zu fördern wäre. Da mit einer gegebenen Pumpleistung bei der Verwendung von Strömungsbeschleunigern mehr Flüssigkeit gefördert werden kann, wird auch in vielen Fällen die damit verbundene Energieeinsparung einen technischen Vorteil bringen. Schließlich kann man, wenn man die Durchsalzmenge nicht vergrößern will, bei Verwendung von SB den Druckverlust herabsetzen oder die Rohre mit kleinerem Querschnitt verwerden. Beides sind Maßnahmen, die die Wirtschaftlichkeit im Betrieb einer Rohrleitung verbessern können.

Als Strömungsbeschleuniger für Wasser oder wässrige Lösungen sind neben hochmolekularen Verbindungen, wie Polyethylenoxid und Polyacrylamid, Lösungen von einigen Tensiden bekannt. Zusätze hochmolekularer Verbindungen besitzen jedoch nur eine beschränkte praktische Einsatzfähigkeit als Strömungsbeschleuniger, da sie in Bereichen hoher Scher- und Dehnbeanspruchung, wie z. B. in Pumpen oder in geringem Maß in der turbulenten Grenzschicht nahe der Bohrwandung, durch mechanischen Abbau irreversibel ihre Wirksamkeit als Strömungsbeschleuniger verlieren. Für geschlossene Wasserkreisläufe wie Kühlkreisläufe und Fernwärmenetze, in denen dieselbe wässrige Lösung durch ein Rohrleitungssystem ständig umgepumpt wird, sind hochmolekulare Zusätze folglich ungeeignet, da der irreversible mechanische Abbau eine laufende Nachdosierung mit wirksamer hochmolekularer Substanz erforderlich macht. Weiterhin ist bekannt, daß Zusätze hochmolekularer Verbindungen ihre Wirksamkeit als Strömungsbeschleuniger oberhalb 90°C verlieren und sie auch aus diesem Grund für Fernwärmenetze ungeeignet sind.

Zusätze von Tensiden in Wasser weisen bekanntlich nicht den Nachteil des irreversiblen mechanischen Abbaues auf (US-PS 3 961 639). Zwar läßt sich auch hier in Bereichen sehr hoher Dehn- und Scherbeanspruchung, wie z. B. in Pumpen, ein mechanischer Abbau beobachten, der jedoch völlig reversibel ist, sobald die Lösung diese Bereiche passiert hat. So ist die strömungsbeschleunigende Wirkung einer wässrigen Lösung von Na-Oleat bei Zusatz von KCl + KOH oder NaCl + NaOH von Savins beschrieben (Rheol. Acta 6, 323 (1967)). Asslanow et al. (Izv. Akad. Nauk. SSSR, Mekh. Zhidk. Gaza 1,36-43 (1980)) untersuchten u.a. wässrige Lösungen von Na-Laurat, -Myristat, -Palmitat und -Stearat bei pH = 11 als SB.

Chang et al. (US-PS 3 961 639) beschrieben die strömungsbeschleunigende Wirkung wässriger Lösungen einiger nicht ionischer Tenside mit Fremdelektrolytzusatz bei Temperaturen im Bereich des Trübungspunktes.

Wesentliche Nachteile der genannten Tensidlösungen sind ihre relative hohen Einsatzkonzentrationen von mindestens 0,25 Gew.-%, die Bildung unlöslicher Seifen mit $Ca^{2+}$ und anderen Kationen, die Ausbildung zweier Phasen die sich bei längerem Stehen trennen und zu Verstopfungen führen können, die Notwendigkeit der Zugabe von korrosionsfördernden Fremdelektrolyten, sowie ein sehr enger Temperaturbereich von wenigen Grad Celsius, in dem SB-Wirkung auftritt. Von diesen Nachteilen nicht behaket sind wäßrige Lösungen einiger kationischer Tenside, wie z.B. Cetylpyridiniumbromid (Inzh. Fizh. Zh. 38, No. 6, 1031 - 1037 (1980) oder Cetyltrimethylammoniumbromid (Nature 214, 585 - 586 (1967)) in jeweils 1 : 1 molarer Mischung mit α-Naphthol. Neben der schlechten Wasserlöslichkeit des α-Naphthols ist hier als entscheidender Nachteil zu nennen, daß derartige Mischungen ihre Wirksamkeit als SB innerhalb weniger Tage durch chemischen Abbau verlieren (US-PS 3 961 639, Conference Proceeding : Intern. Conference on Drag Reduction, 4.-6.9.1974 Rolla Missouri, USA). Ein weiterer Nachteil aller bisher bekannten Tensidlösungen ist, daß sie oberhalb 90°C ihre Wirksamkeit als SB verlieren, und daß sie somit völlig ungeeignet sind für Fernwärmenetze.

Die para-Toluolsulfonate von N-Dodecylpyridinium, Ethyltrimethylammonium und N-Octadecylpyridinium sind bereits bekannt aus Chemical Abstracts 56, 11109 i ; 95, 175 879 g und 58, 11376 e. Sie werden als Emulgatoren in photographischen Emulsionen eingesetzt. Ebenfalls bekannt sind die Salicylate, Halogenbenzoate und Alkylcarboxylate von $C_{16}$-Alkyl- und $C_{16}$-Alkenyl-trialkylammonium- bzw. pyridinium-Salzen und deren Verwendung als Strömungsbeschleunigen (EP-A-91 086 ; Stand der Technik nach Art. 54 (3) EPÜ).

Überraschenderweise wurde nun gefunden, daß zum Unterschied zu allen bisher als SB bekannten Tensiden die nachfolgend aufgeführten Verbindungen in reiner Form auch ohne jegliche Zusätze in wäßriger Lösung schon in kleinsten Konzentrationen als Strömungsbeschleuniger wirksam sind. Weiterhin wurde gefun-

den, daß einige dieser Verbindungen auch bei Temperaturen oberhalb 90°C selbst bei Dauerbeanspruchung über Wochen als SB wirksam bleiben und keine Abnahme ihrer Effektivität zeigen.

Gegenstand der Erfindung sind neue quartäre Ammoniumsalze der Formel

$$R_1\text{-}K^{\oplus}\ A^{\ominus}$$

wobei $R_1$ $C_{12}$ - $C_{26}$-Alkyl oder $C_{12}$ - $C_{26}$- Alkenyl, $K^{\oplus}$ eine Gruppe der Formel

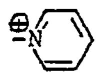 oder $-^{\oplus}N(R_2)_3$, $R_2$ $C_1$ - $C_3$-

Alkyl, vorzugsweise Methyl und $A^{\ominus}$ ein Anion der folgenden Formeln bedeutet : $R_3SO_3^{\ominus}$, wo $R_3$ $C_6$ - $C_9$-Alkyl oder Alkenyl ist und die Summe der C-Atome in $R_1$ und $R_3$ mindestens 21 betragen soll ;

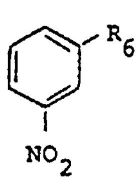 ; 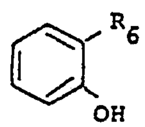 ; 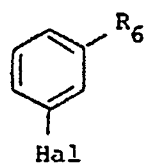 ; 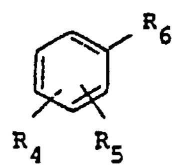 oder

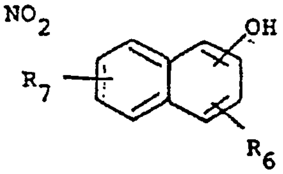

wo Hal Fluor Chlor, Brom oder Jod $R_4$ $C_1$ - $C_5$-Alkyl, $C_2$-$C_5$-Alkenyl oder $C_1$ - $C_5$-Alkoxy in den Stellungen 3, 4, 5 und 6, $R_5$ Wasserstoff oder Hydroxy in den Stellungen 2 und 3, $R_6$ $COO^{\ominus}$ oder für den Fall $R_5$ = OH auch $SO_3^{\ominus}$ und $R_7$ Wasserstoff oder Methyl ist, mit der Maßgabe, daß A nicht ein Salicylat- oder m-Halogenbenzoat-Ion ist, wenn $R_1$-$K^+$ $C_{16}$-Alkyl- pyridinium, $C_{16}$-Alkenyl-pyridinium, $C_{14}$-$C_{24}$-Alkyl tri- $C_1$-$C_3$ alkylammonium und $C_{18}$-Alkenyl-tri $C_1$-$C_3$ alkylammonium bedeutet.

Besonders bevorzugt sind die Salze aus den folgenden Kationen und Anionen :

1. $[C_nH_{2n+1}N(CH_3)_3]^{\oplus}$ oder 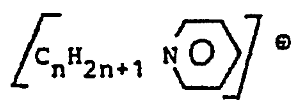

a) mit dem Anion $C_6H_{13}SO_3^{\ominus}$ für $20 \leqq n \leqq 26$
b) mit dem Anion $C_7H_{15}SO_3^{\ominus}$ für $14 \leqq n \leqq 22$
c) mit dem Anion $C_8H_{17}SO_3^{\ominus}$ für $14 \leqq n \leqq 20$

2. $[C_nH_{2n+1}N(CH_3)_3]^{\oplus}$ oder 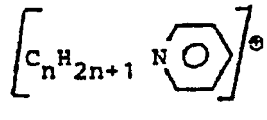

für $12 \leqq n \leqq 24$ mit folgenden Säureanionen

a) 2-Phenol-sulfonat, Salicylat oder m-Halogenbenzoat mit Ausnahme von n = 14 - 24 für die beiden letztgenannten Anionen in Verdindung mit dem ersten Kation und n = 16 in Verbindung mit dem zweiten Kation,

b) 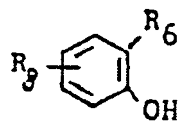

mit $R_8$ = Methyl oder Ethyl oder Propyl oder $C_nH_{2n+1}O$ mit $1 \leqq n \leqq 4$, vorzugsweise in den Stellungen 3 oder 4 oder 5 zur Carboxylgruppe.

c) 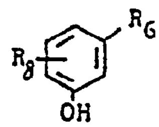

mit $R_8$ = Methyl oder Ethyl oder Propyl oder $C_nH_{2n+1}O$ mit $1 \leqq n \leqq 4$, vorzugsweise in den Stellungen 4 oder 5 zur Carboxyl-Gruppe.

d) 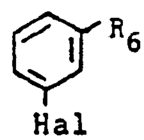

mit Hal = F, Cl, Br, J

3. $[C_nH_{2n+1}N(CH_3)_3]^{\oplus}$ oder 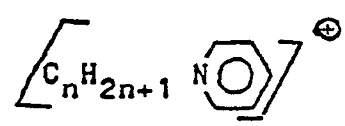

für $12 \leqq n \leqq 24$
mit den Anionen 2-Hydroxy-1-naphthoat, 3-(oder 4)-Hydroxy-2-naphthoat bzw. die entsprechenden Derivate der Naphthol-Sulfonsäuren.

Die Herstellung dieser neuen quartären Ammoniumsalze kann auf folgenden Wegen erfolgen.

Man löst zunächst die Alkyl-trimethylammonium-halogenide bzw. Pyridinium- chloride, -bromide oder auch -jodide in wasserfreien Lösungsmitteln wie z. B. Methanol auf und gibt frisch gefälltes, zuletzt mit Methanol gewaschenes Silberhydroxyd in leichtem Überschuß hinzu. So soll möglichst kein Wasser in das System eingebracht werden, da man sonst Schwierigkeiten mit der Kristallisation bekommt. Bei kurzem Anwärmen auf ca. 50° vollzieht sich die Bildung des Alkyltrimethyl-ammonium- bzw. Pyridiniumhydroxyds, das im Methanol gelöst bleibt.

Bei dieser Umsetzung verschwindet die braune Farbe des Silber-hydroxyds weitgehend und der resultierende Niederschlag nimmt die Farbe des Silberhalogenids an. Der Silberhalogenid-Niederschlag wird dann bei ca. 15° abesaugt. Das im methanolischen Filtrat vorliegende Alkyltrimethylammonium- bzw. Pyridiniumhydroxyd kann durch Zugabe der stöchiometrischen Menge einer Carbonsäure oder Sulfosäure neutralisiert werden. Durch Eindampfen des Methanols wird das gewünschte Alkyl-trimethylammoniumsalz bzw. Pyridiniumsalz gewonnen.

Noch einfacher ist es, zunächst die Silbersalze der betreffenden Carbonsäure herzustellen, die häufig wenig wasserlöslich sind. Man kann hier von Silberhydroxyd oder Silbercarbonat ausgehen und diese mit der gewünschten Carbonsäure neutralisieren. Man kann aber auch die Alkalicarboxylate in Wasser lösen und Silbernitrat-Lösung zugeben, wenn das Silbercarboxylat ausfällt. Man saugt ab, wäscht und trocknet das Silbercarboxylat. Man kann dieses dann in der stöchiometrisch erforderlichen Menge zu einer Lösung des Alkyl-trimethylammonium- bzw. Pyridiniumhalogenids in einem wasserfreien Lösungsmittel wie Methanol geben und kurz auf 50 bis 60°C erwärmen.

Auch hier wird hinterher bei 15°C das Silberhalogenid abgesaugt und durch Eindampfen des Filtrats das gemischte Alkyl-trimethylammonium- bzw. Pyridiniumcarboxylat gewonnen. Eine weitere Reindarstellung kann durch Umkristallisieren aus prakt. wasserfreien Lösungsmitteln (Essigester, Aceton, Acetonitril, Dichlorethan) erfolgen.

Als weitere Möglichkeit ist auch die Gewinnung der Alkyltrimethylammonium- bzw. Pyridiniumhydroxyd-Lösung durch Behandlung der Alkyl-trimethylammonium- bzw. Pyridiniumhalogenide (oder anderer Salze) mit einem stark basischen Anionenaustauscher zu erwähnen, was ebenfalls in einem wasserfreien Lösungsmittel wie z. B. Methanol geschehen muß. Daran schließt sich wiederum die Neutralisation mit der gewünschten Carbonsäure an.

Die genannten Salze eignen sich zur Verminderung des Reibungswiderstandes von wässrigen Medien. Sie werden zugegeben in Konzentrationen von 0,006 bis 2 Gew.-%, vorzugsweise 0,04 bis 0,4 Gew.-%, wobei jedoch für jedes Salz eine andere untere kritische Konzentrationsgrenze für eine ausreichende Wirksamkeit als SB existiert. Die Wirkung als SB is außerdem abhängig von der Temperatur. Je nach eingesetztem Salz wird eine austeichende Wirkung als SB im Temperaturbereich von 0°C bis 90°C und für einige, im folgenden noch aufgeführte Tenside wurde erstmals Reibungsminderung auch über 90°C gefunden. Die untere Temperaturgrenze für einen Einsatz als SB ist bei allen Tensiden die Löslichkeitstemperatur. Ist das Tensid jedoch in Lösung, so kann die Löslichkeitstemperatur einige Stunden bis Wochen, um 5 - 20°C unterschritten werden.

Für n-Dodecyltrimethylammoniumsalicylat wurde folgender Bereich, in dem die optimale Wirksamkeit als SB auftritt, gefunden : 0,14 bis 1 Gew.-% von 0 bis 30°C. Allgemein gilt, daß der Temperaturbereich, in dem eine ausreichende Wirkung als SB vorliegt, mit jeder zusätzlichen-$C_2H_4$-Gruppe um ca. 15°C zu höheren Temperaturen hin verschoben wird. Bei Verwendung der Pyridiniumverbindungen liegt der bevorzugte Temperaturbereich um ca. 8 - 12°C niedriger gegenüber den Trimethylammonium-Salzen gleicher Kettenlänge.

Der bevorzugte Temperatur- und Konzentrationsbereich wird aber nicht nur durch die Kettenlänge der n-Alkyltrimethylammonium- oder n-Alkylpyridiniumverbindung bestimmt, sondern auch durch die Art des Gegenions.

So zeigen z. B. die Verbindungen Hexadecyltrimethylammonium-3-(oder 4)-methylsalicylat oder Hexadecyltrimethyl-4-ethoxy-salicylat in wässriger Lösung bei 40°C bereits ab 0,006 Gew.-% bis 1 Gew.-% und oberhalb 65°C im Konzentrationsbereich 0,05 bis 1 Gew.-% eine ausreichende Wirkung als SB, während Hexadecyltrimethylammoniumsalicylat bei 30°C ab 0,015 Gew.-% und oberhalb 70°C nur bei Konzentrationen größer 0,8 Gew.-% eine Wirksamkeit als SB aufweist.

Ganz allgemein zeigen die Verbindungen, n-Alkyltrimethylammonium und n-Alkylpyridinium mit den Gegenionen

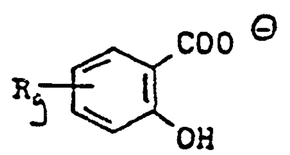

oder

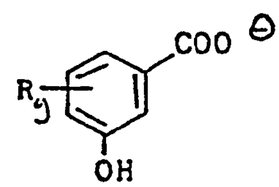

mit $R_9$ = $C_1$ - $C_5$ Alkyl oder $C_1$ bis $C_5$ Alkoxy mit zunehmender Kettenlänge von R eine Verschiebung der unteren Konzentrationsgrenze für eine Wirksamkeit als SB zu kleineren Werten und gleichzeitig eine Ausweitung des Temperaturbereiches zu höheren Werten verglichen mit den entsprechenden reinen Salicylat-Verbindungen. Eine weitere Erhöhung des Temperaturbereiches, in dem eine Wirkung als SB vorliegt, wird durch Verwendung der Tenside mit den Anionen

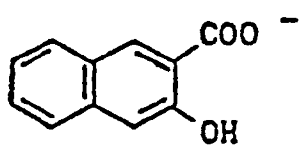

erreicht.

Beispielsweise zeigen die Verbindungen Hexadecyltrimethyl-ammonium-3(oder 4) hydroxy-2-naphthoat Wirksamkeit als SB bei 50°C im Konzentrationsbereich von 0,005 Gew.-% bis 1 Gew.-% und oberhalb 80°C im Konzentrationsbereich von 0,025 Gew.-% bis 1 Gew.-%. Analoges gilt für die entsprechenden Pyridiniumverbindungen, nur daß die Temperaturbereiche im Mittel um ca. 5 - 15°C nach unten verschoben sind Analog

zu den Salicylat-Verbindungen bewirkt auch bei den Hydroxynaphthoaten die Veränderung der Kettenlänge des n-Alkyltrimethylammonium- und n-Alkylpyridiniumkations eine Verschiebung des Temperaturbereiches für die SB-Wirkung. Beispielsweise zeigen jeweils 1000 ppm-Lösungen der Verbindungen n-$C_nH_{2n+1}$trimethylammonium-3-hydroxy-2-naphthoat ab Temperaturen von 65 - 85°C mit n = 18 auch oberhalb 105°C, mit n = 20 auch oberhalb 115°C und mit n = 22 auch oberhalb 125°C Wirksamkeit als SB. Ganz allgemein wird bei den n-Alkyltrimethylammonium- bzw. n-Alkylpyridinium-3-hydroxy-2-naphthoaten der wirksame Temperaturbereich um 30 bis 40°C zu höheren Temperaturen hin erweitert im Vergleich zu den jeweiligen n-Alkyltrimethylammonium- bzw. n-Alkylpyridinium-salicylaten.

Ähnliche Zusammenhänge zwischen den Stukturen der Tensidsalze und den wirksamen Temperatur- und Konzentrationsbereichen gelten für die jeweils entsprechenden Arylsulfonate. Häufig treten jedoch höhere Löslichkeitstemperaturen als bei den entsprechenden Arylkarbonsäuren auf. So läst sich z.B. n-Hexadecyltrimethylammonium-2-phenolsulfonat nicht wie das entsprechende Salicylat bereits ab 30° C, sonderen erst bei Temperaturen oberhalb 50°C in Wasser. Wirksamkeit als Strömungsbeschleuniger liegt für das 2-Phenolsulfonat daher im Temperaturbereich von 50°C bis 70°C bei Konzentrationen von 0,05 bis 2 Gew.% vor, während das entsprechende Salicylat bereits ab 30°C Wirksamkeit zeigt.

Bei den n-Alkyltrimethylammonium-n-alkyl-1-sulfonaten und n-Alkylpyridinium-n-alkyl-1-sulfonaten wird der Temperaturbereich und Konzentrationsbereich in dem Wirksamkeit als SB vorliegt, ebenfalls durch die Kettenlängen der Alkylgruppen sowohl beim Anion als auch beim Kation bestimmt.

Beispielsweise zeigt die Verbindung Tetradecyltrimethyl-ammonium-heptan-1-sulfonat im Konzentrationsbereich von 0,02 bis 2 Gew.-% vorzugsweise 0,07 bis 0,5 Gew.-%, im Temperaturbereich von 0°C bis 38°C Wirksamkeit als SB. Die Verbindung Hexadecyltrimethylammonium-heptan-1-sulfonat besitzt hingegen im Konzentrationsbereich von 0,02 bis 2 Gew.-%, vorzugsweise 0,07 bis 0,5 Gew.-% im Temperaturbereich von 10°C bis 50°C Wirksamkeit als SB.

Eine weitere Erhöhung der Kettenlänge des n-Alkyltrimethylammonium- oder n-Alkylpyridinium-Kations bewirkt eine weitere Verschiebung des wirksamen Temperaturbereiches zu höheren Temperaturen. Beispielsweise zeigt die Verbindung n-Docosyltrimethylammonium-n-heptan-1-sulfonat im Konzentrationsbereich 0,1 bis 1 Gew.-% auch für Temperaturen oberhalb 75°C noch SB-Wirkung.

Im Vergleich zu den n-Heptan-1-sulfonaten besitzen die jeweils entsprechenden n-Octan-1-sulfonate Wirksamkeit als SB in einem um 10 bis 30°C höheren Temperaturbereich. Beispielsweise zeigt die Verbindung n-Tetradecyltrimethyl-ammonium-n-octan-1-sulfonat im Konzentrationsbereich 0,2 bis 1 Gew.-% auch oberhalb 45°C Wirksamkeit als SB und die Verbindung n-Octadecyltrimethylammonium-n-octan-1-sulfonat im Konzentrationsbereich von 0,2 bis 1 Gew.-% auch oberhalb 85°C noch Wirksamkeit als SB. Allgemein bewirkt die Verlängerung der n-Alkylkette sowohl bei den Anionen als auch den Kationen eine Verschiebung des wirksamen Temperaturbereiches zu höheren Temperaturen hin.

Bei den genannten Tensiden mit den Anionen $R\text{-}SO_3^{\ominus}$, insbesondere bei den n-Octan-1-sulfonaten und n-Nonan-1-sulfonaten, ist bis 100°C häufig keine klare Lösung erhältlich ; die Wirkung als SB wird hierdurch nicht beeinträchtigt.

Von allen genannten Tensiden eignen sich als SB über 90°C insbondere die Salze $C_nH_{2n+1}K^{\oplus} A^{\ominus}$, wobei $K^{\oplus}$ für die quartäre Stickstoffgruppe steht

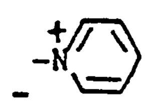 oder $-\overset{+}{N}(R)_3$

mit $R = -CH_3$ oder $-C_2H_5$ und A die folgenden Anionen beinhaltet :

1. für n = 22 bis 26 Salicylat, 3-Halogen-benzoat mit Halogen = F, Cl, Br, J
2. für n = 20 bis 26 5 oder 6-Methyl-salicylat, Methoxy-salicylat, n-Octan-1-sulfonat
3. für n = 18 bis 26 3- oder 4-Methyl-2- oder 3-Hydroxybenzoat, 3- oder 4-Ethoxy-2- oder 3-Hydroxybenzoat, n-Nonan-1-sulfonat, 2-Hydroxy-1-naphthoat
4. für n = 16 bis 28 3- oder 4-Alkyl-2- oder 3-hydroxybenzoat, mit Alkyl = $C_2$ - $C_4$, 3- oder 4-Alkoxy-2- oder 3-hydroxybenzoat mit Alkyl = $C_3$ - $C_5$ und 3- oder 4-Hydroxy-2-naphthoat.

Wirksam als SB für Temperaturen über 100°C sind insbesondere die Salze der Gruppe 4 für n $\geqq$ 18 ; nach Beispiel 23 zeigt die Verbindung $/C_{22/20}H_{41/45}N(CH_3)_3/$. 3-Hydroxy-2-naphthoat noch bei 120°C bis 130°C eine sehr gute Wirksamkeit als SB bei einer Konzentration von 814 ppm bis 1000 ppm.

Weiter wurde gefunden, daß durch Erhöhung des pH-Wertes der wässrigen Lösung auf pH-Werte über 8, vorzugsweise auf pH 9 bis 10,5, durch Zugabe von NaOH oder anderer Basen oder durch Zugabe von $Na_2CO_3$ oder anderer Salze, die den pH-Wert erhöhen, die Wirksamkeit als SB entweder nicht beeinflußt wird, wie z. B. bei den Sulfonaten, oder wesentlich verbessert wird, wie z. B. bei den Hydroxy-benzoaten und den davon

abgeleiteten Verbindungen. Auch eine Absenkung des pH-Wertes mit HCl oder anderen starken Säuren auf pH-Werte unter 4,5 führt zu der gleichen Beeinflussung der SB-Wirkung der Tenside.

Die Zugabe anderer Fremdelektrolyte führt von keiner Beeinflussung, wie z. B. bei den Sulfonaten, bis zu einer Verbesserung der Wirkung als SB, wie z. B. bei den Hydroxybenzoaten und den davon abgeleiteten Verbindungen.

Als solche Fremdelektrolyte kommen in Frage beispielsweise schwache Säuren wie Essigsäure oder Ameisensäure und Salze, die aus den folgenden Ionen gebildet werden : Alkali-, Erdalkali-, Übergangsmetall-, Ammonium- oder Aluminium-Kationen ; Halogenide,

$C10_3^{\ominus}$, $C10_4^{\ominus}$ $BrO_3^{\ominus}$ $JO_3^{2\ominus}$ $SO_2O_3^{2\ominus}$ $SO_4^{2\ominus}$, $S_2O_8^{2\ominus}$, $NO_2^{\ominus}$, $B_4O_7^{2\ominus}$, $NO_3^{\ominus}$, $PO_4^{3\ominus}$, $CO_3^{2\ominus}$, $CH_3COO^{\ominus}$, $CH_3COO^{\ominus}$, $C_2O_4^{2\ominus}$, $CN^{\ominus}$, $CrO_4^{2\ominus}$, $Cr_2O_7^{2\ominus}$. Die Menge dieser Fremdelektrolyte, die man der wässrigen Tensidlösung zugeben kann, ist nach oben begrenzt durch die Konzentration, bei der ein Aussalzeffekt für das Tensid auftritt verbunden mit einer Abnahme oder dem völligen Verschwinden der Wirksamkeit als SB.

Die Wirkung der Fremdelektrolyte hängt auch ab von der Wertigkeit der Ionen und zwar verschiebt sich die Wirkung zu geringeren Konzentrationen gemäß folgendem Schema : 1-1-wertiger Elektrolyt < 2-1 wertiger Elektrolyt < 1-2-wertiger Elektrolyt < 2-2-wertiger Elektrolyt < 3-2-wertiger Elektrolyt < 2-3-wertiger Elektrolyt. Die Verbesserung der Wirksamkeit als Strömungsbeschleuniger bei den Hydroxybenzoaten und den daraus abgeleiteten Verbindungen ist besonders effektiv bei Zugabe eines Salzes, das gleichzeitig den pH-Wert auf pH $\geqq 9,9$ anhebt. So wirkt sich z. B. die Zugabe von $Na_2CO_3$ im Konzentrationsbereich 0,1.C bis 10°C besonders positiv aus, wenn C die molare Konzentration des eingesetzten Tensids ist.

Anstelle der Zugabe von Salzen kann man auch so vorgehen, daß man das Halogensalz des kationischen Tensids $R_1K^+$ Hal-, wie z. B.

$$\left[C_nH_{2n+1}N(CH_3)_3\right]Hal$$ oder 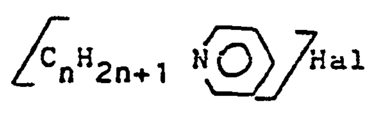

Hal mit Hal = Cl, Br, J im molaren Verhältnis 1 : 1 mit einem Alkalisalz des Anions NaA, wie z. B. Na-n-alkyl-1-sulfonat, Na-hydroxy-benzoat und die davon abgeleiteteten Säureanion oder wie z. B. Na-hydroxy-naphthoat als Strömungsbeschleuniger einsetzt. Die Wirkung ist dann gleich der Wirkung, die man mit den reinen Tensidsalzen unter Zusatz von Alkalihalogeniden erreicht. Auch Mischungen die vom molaren Verhältnis 1 : 1 abweichen, wie z. B. 1 : 2, zeigen noch Wirkung als SB. Die maximale Wirksamkeit als Strömungsbeschleuniger ist auch von der Zeit abhängig, die verstrichen ist seit der Herstellung der wässrigen Tensidlösungen. Die Tensidlösungen zeigen zwar bereits sofort nach dem Ansetzen der Lösungen eine Wirkung als Strömungsbeschleuniger, doch kann sich diese Wirkung während einer Woche noch deutlich ändern. Die Zeit, die benötigt wird, um eine optimale Wirkung zu erzielen, läßt sich für den Einzelfall unschwer durch einfache Versuche ermitteln. In den meisten Fällen wird die optimale Wirkung nach einer Woche erreicht. Eine Änderung oder Verbesserung der Wirkung tritt dann nicht mehr ein.

Von einigen Tensiden, wie z. B. dem Hexadecylpyridiniumsalicylat ist bekannt (H. Hoffmann et al., Ber. Bunsenges. Phys. Chem. 85 (1981) 255), daß sie ab einer ganz bestimmten, für jedes Tensid charakteristischen Konzentration, der $CMC_{II}$, große, nichtkugelförmige, meist stäbchenförmige Mizellen aus den einzelnen Tensidionen und Gegenionen aufbauen.

Überraschenderweise wurde nun gefunden, daß Tenside in wässriger Lösung immer dann als Strömungsbeschleuniger wirksam sind, wenn sie für Konzentrationen größer der $CMC_{II}$ nichtkugelförmige, vorzugsweise stäbchenförmige Mizellen ausbilden. Nichtkugelförmige, vorzugsweise stäbchenförmige Mizellen liegen vor, wenn bei der Untersuchung der Tensidlösung mit Hilfe der Methode der elektrischen Doppelbrechung mit gepulstem, rechteckförmigen elektrischen Feld (E. Frédericq und C. Houssier, Eletric Dichroism and Eletric Birefringence, Claredon Press, Oxford 1973 und H. Hofmann et al., Bar. Bunsenges. Phys. Chem. 85 (1981) 255) ein Meßsignal gefunden wird, aus dessen Abfall sich eine Relaxationszeit von $\tau \geqq 0,05$ µs bestimmen läßt. Die untere Konzentrationsgrenze, ab der ein Tensid in wässriger Lösung als Strömungsbeschleuniger wirksam ist, wird daher immer durch die $CMC_{II}$, vorzugsweise durch den 1,5-fachen Konzentrationswert der $CMC_{II}$, festgelegt. Die Bestimmung der $CMC_{II}$ ist z. B. durch Messung der elektrischen Leitfähigkeit der Tensidlösung in Abhängigkeit von der Tensidkonzentration möglich, wie bei H. Hoffmann et al.(Ber. Bunsenges. Phys. Chem. 85 (1981) 255) beschrieben. Es zeigte sich, daß der Wert der $CMC_{II}$ temperaturabhängig ist und sich mitzunehmender Temperatur zu höheren Tensidkonzentrationen veschiebt.

Zur Festlegung der Tensidkonzentration, die minimal notwendig ist, um eine ausreichende Wirkung als SB in einem bestimmten Temperaturbereich zu erzielen, ist die Bestimmung der $CMC_{II}$ bei der Anwendungstemperatur mit Hilfe der elektrischen Leitfähigkeit ein geeigneter Vorversuch.

Die Untersuchung der genannten Tenside auf ihre Wirksamkeit als SB erfolgte in den meisten Fällen in der üblichen Art, indem für die jweilige wässrige Lösung der Tenside der Druckabfall $\Delta P$ über die Strecke L bei Durchströmen eines Rohres mit dem Querschnitt d für verschiedene Strömungsgeschwindigkeiten u gemessen wurde. Aus diesen Werten lassen sich die dimensionslosen Größen, Reibungsbeiwert $\lambda$ und die Reynoldszahl Re, berechnen,

$$\lambda = \frac{2\,d}{\varsigma\, u^2} \cdot \frac{\Delta P}{L}$$

wobei $\delta$ die Dichte und $\gamma$ die kinematische Viskosität bedeuten. Üblicherweise werden für $\delta$ und $\gamma$ jeweils die entsprechenden Werte des reinen Lösungsmittels, Wasser, eingesetzt. Die so erhaltenen Werte, $\lambda$, Re, für die untersuchten Tensid-Lösungen wurden in der üblichen doppelt logaritmischen Auftragung gegen Re mit den entsprechenden Werten für reines Wasser, wiedergegeben durch

$$1/\sqrt{\lambda} = 2 \log Re\sqrt{\lambda} - 0{,}8$$

verglichen. Eine Wirkung als SB oder auch Reibungsminderung liegt dann vor, wenn gilt: $\lambda H_2O - \lambda_{SB} > 0$, bzw. die Größe der Reibungsminderung in Prozent berechnet sich nach:

$$\alpha = \%\ \text{Reibungsminderung} = \frac{\lambda H_2O - \lambda SB}{\lambda H_2O} \times 100$$

Wie aus Figur 1 ersichtlich ist, wirken die genannten Tensidlösungen als SB in der Weise, daß die prozentuale Reibungsminderung mit ansteigender Reynoldszahl zunimmt, dann aber nach Überschreiten einer bestimmten Reynoldszahl, $Re_{max}$, mit maximaler prozentualer Reibungsminderung, sehr schnell wieder abnimmt. Der Grad der Wirksamkeit einer Tensidlösung als SB soll im folgenden Text durch die Größe von $Re_{max}$ gekennzeichnet werden ; demnach besitzt einer Tensidlösung mit $Re_{max}$ = 20 000 eine bessere Wirksamkeit als SB als eine Tensidlösung mit $Re_{max}$ = 10 000. Der zugehörige $\alpha$-Wert soll mit $\alpha_{max}$ gekennzeichnet werden. Die Untersuchungen der Tensidlösungen ergaben meistens nur dann reproduziarende Ergebnisse, wenn die wässrigen Lösungen der Tensidsalze vor den Messungen jeweils für ca. 1 Woche bei den Meßtemperaturen aufbewahrt wurden. Die Lösungen zeigen zwar auch sofort nach dem Ansetzen eine Wirkung als Strömungsbeschleuniger, die sich jedoch im Verlauf einer Woche noch deutlich ändern kann.

Die so bahandelten Tenside wurden einer Vielzahl von Tests unterzogen. So ergaben Dauerversuche über viele Tage, wie aus Beispiel 18 ersichtlich, daß bei den aufgeführten Tensiden keine Abnahme ihrer strömungsbeschleunigenden Wirkung durch mechanischen oder chemischen Abbau auftritt. Weiterhin zeigte sich, daß die Wirksamkeit als SB der genannten Tenside mit zunehmender Konzentration zunimmt ; allerdings steigt auch die Viskosität der Lösungen an, so daß die prozentuale Reibungsminderung bei kleineren Reynoldszahlen schlechter wird, wie aus Figur 1 zu entnehmen ist.

Die durchgeführten Untersuchungen zeigen, daß sich die genannten Tensid-Salze als Strömungsbeschleuniger überall dort eignen, wo Wasser durch Rohrleitungen gepumpt wird, insbesondere aber dort, wo Wasser in einem Rohrleitungssystem ständig im Kreislauf umgepumpt wird, wie z. B. in Kühlkreisläufen, da hier ein hohe Langzeitstabilität das SB, wie sie die genannten Tensid-Salze aufweisen, unbedingt erforderlich ist. Zusätzlich zeigen einige der genannten Tenside eine Eignung insbesondere für Fernwärmenetze, da diese Tenside auch oberhalb von 90°C bei Beanspruchung über Wochen (siehe Beispiel 22) ihre Wirkung als Strömungsbeschleuniger beibehalten.

Die Zudosierung der Tensid-Salze in das die Rohrleitungen durchströmende Wasser kann sowohl in Form einer konzentrierten Tensidlösung (1 - 10 Gew.-%) erfolgen, als auch durch Zugabe der reinen kritallinen Tensid-Salze. Wegen des guten Durchmischungseffektes ist eine Zudosierung in das Rohrleitungssystem kurz vor einer Pumpe der günstigste Ort.

**Beispiel 1**

42,0 g Silbernitrat werden in 175 g Wasser aufgelöst. Unter Rühren wird diese Silbernitrat-Lösung in eine aus 11,5 g Natriumhydroxid (99 %ig) und 35 g Wasser bereitete Natronlauge eingerührt. Die entstandene Silberhydroxyd-Fällung wird abgesaugt, viermal mit Wasser, dann viermal mit Methanol gewaschen. Man gibt das (methanolfeuchte) Nutschgut zu einer Lösung von 72,9 g Cetyl-trimethylammoniumbromid (käuflich) in 600 ml Methanol. Man erwärmt kurz (2 Min.) auf 60°, kühlt auf ca. 10° ab und saugt das entstandene Silberbromid ab. Das Filtrat ist eine wasserhelle, klare Lösung von Cetyl-trimethylammoniumhydroxyd in Methanol. Man teilt sie in drei gleiche Teile und neutralisiert das erste Drittel durch Zugabe von 10,29 g m-Kresotinsäure [4-Methyl-2-oxy-bezoesäure]. Nach dem Eindampfen der Methanollösung am Rotationsverdampfer erhält man 29,0 g farbloses Pulver, das durch wiederholte Umkristallisation aus (400 ml) Essigester gereinigt werden kann: Farblose Blättchen.

Diese Verbindung kann mit Perchlorsäure in Eisessig titriert werden.

Beispiel 1a und 1b

In gleicher Weise kann das Cetyltrimethylammoniumhydroxyd auch mit der 3-Methyl- (1 a) und mit der 5-Methyl-2-oxy-benzoesäure (1b) neutralisiert werden. Auch diese Substanzen werden durch Umkristallisieren aus Essigester in Form farbloser Blättchen erhalten.

**Beispiel 2**

Ein zweites Drittel der Cetyl-trimethylammonium-hydroxy-Lösung kann durch Zugabe von 12,71 g 3-Oxy-2-naphthoesäure (titr. ÄG 190,6) neutralisiert werden. Nach Einengen dieser Lg. im Rotationsverdampfer zur Trockne erhält man 30,5 g eines leicht bräunlichen Pulvers.

Durch Umkristallisation aus 300 ml Aceton bekommt man das 3-Oxy-2-naphthoat als hellgelbe Kristalle (Tritation mit Perchlors. in Eisessig).

ÄG = Äquivalentgewicht

Cetyl = Hexadecyl

**Beispiel 3**

Das letzte Drittel der Cetyl-trimethylammoniumhydroxyd-Lösung wird mit 12,39 g 4-Äthoxy-2-oxy-benzoesäure (deren Äquivalentgewicht durch Titration mit 0,1 n Natronlauge zu 185,8 ermittelt worden war) durch Einstreuen als Pulver neutralisiert. Die erhaltene gelbe Lösung in Methanol wurde eingeengt bis zur Trockne. So erhielt man 31,1 g eines leicht bräunlichen Rückstandes, der durch mehrmaliges Umkristallisieren aus 300 ml Essigester gereinigt werden konnte. Man erhält das Cetyl-trimethylammonium-4-ethoxy-salicylat in (leicht rötlichen) Kristallen (Titration mit Perchlorsäure in Lösung ergibt das erwartete Äquivalentgewicht).

**Beispiel 4**

24,5 g Heptan-1-sulfonsäure Na-monohydrat werden in 35 g Wasser aufgelöst. Man rührt diese Lösung in eine Lösung von 18,1 g Silbernitrat in 25 g Wasser ein. Das sich abscheidende Silbersalz wird abgesaugt und zweimal mit wenig Methanol gespült. Man erhält nach dem Trocknen 26,2 g (85,3 % d. Th.) Silber-Heptan-1-sulfonat als farbloses Kristallpulver.

Zu einer Lösung von 37,74 g Tetradecyl-trimethylammoniumchlorid in 250 ml Methanol werden 37,2 g Siberheptan-1-sulfonat gegeben. Nach kurzem Erwärmen wird das Silberchlorid abgesaugt; das wasserhelle Filtrat wird im Rotationsverdampfer eingeengt: 55,5 g Rohprodukt.

Durch Kristallisation aus 350 ml Dichlorethan erhält man das Tetradecyltrimethylammonium-heptan-1-sulfonat is großen farblosen Blättchen.

**Beispiel 5**

Es wurde eine Konzentrationsreihe von 300, 500, 750, 1000, 1500 Gew.-ppm von Tetradecyltrimethylammonium-heptan-1-sulfonat (abgekürzt: $C_{14}$TA-heptan-1-sulfonat) in E-Wasser angesetzt, indem die entsprechenden Gewichtsmengen von 0,3 ; 0,5 ; 0,75 ; 1,0 und 1,5 g $C_{14}$TA-heptan-1-sulfonat auf 1000 g E-Wasser eingewogen wurden. Während des Auflösens wurden die Lösungen unter Rühren kurz auf ca. 90°C erhitzt und nach dem Abkühlen auf Raumtemperatur (23°C) für 1 Woche ohne zu Rühren bei dieser Temperatur aufbewahrt.

Die Untersuchung auf Reibungsminderung erfolgte anschließend in einem Turbulenzrheometer (Polymer

Letters 9,851 (1971), indem analog zu einer Spritze eine Flüssigkeitsmenge von 1,5 l mit Hilfe eines Kolbens durch das Meßrohr gedrückt wird. Die Bewegung des Kolbens wird während der Messung beschleunigt, so daß die gesamte Fließkurve, wie in der Figur 1 dargestellt, in einer Messung erfaßt wird. Der Durchmesser des Meßrohres beträgt 3 mm, die Meßstrecke für $\Delta P$ 300 mm und die Einlaufstrecke 1200 mm.

In dieser Apparatur wurde dieselbe Konzentrationsreihe von $C_{14}$TA-1-heptan-sulfonat bei 23°C und 35°C gemessen, nachdem die Lösung zuvor ebenfalls für 1 Woche bei 35°C aufbewahrt worden war.

Die Tabellen 1 und 2 fassen die Ergebnisse aller Messungen für 23°C und 35°C durch Wiedergabe von $Re_{max}$ und $\alpha_{max}$ zusammen.

**Beispiel 6**

Unterschiedliche Mengen an $Na_2CO_3$ wurden zusammen mit $C_{14}$TA-heptan-1-sulfonat, wie in Beispiel 6 beschrieben, zu wässrigen Lösungen angesetzt, deren Konzentrationen an $C_{14}$TA-heptan-1-sulfonat jeweils 750 ppm (1,72 mol/l) und an $Na_2CO_3$ (in mol/l) wie folgt gewählt wurden :

$1 . 10^{\ominus 4}$ ; $2 . 10^{\ominus 4}$ ; $1 . 10^{\ominus 3}$ ; $1{,}72 . 10^{\ominus 3}$ ; 0,01 ; 0,1.

Die Ergebnise der Untersuchung auf Reibungsminderung bei 23°C im Turbulenzrheometer sind in Tabelle 3 zusammengefaßt. Wie aus Tabelle 3 ersichtlich bewirkt die $Na_2CO_3$-Zugabe bis 1,72 $10^{-3}$ mol/l keine Beeinflussung, ab dem 10fachen molaren Überschuß eine Verschlechterung der Wirkung als SB für $C_{14}$TA-heptan-1-sulfonat.

**Beispiel 7**

Wässrige Lösungen unterschiedlicher Konzentration an $C_{14}$TA-heptan-1-sulfonat wurden durch Einwiegen der Salze $C_{14}$TA-Cl und Na-heptan-1-sulfonat im molaren Mischungsverhältnis 1 : 1 mit den Gesamtkonzentrationen 500, 1000, 1500 und 2000 ppm, wie in Beispiel 5 beschrieben, hergestellt. Die Ergebnisse der Untersuchung auf Reibungsminderung in Turbulenzrheometer sind in Tabelle 4 zusammengefaßt. Wie aus der Tabelle 4 ersichtlich beeinflußt die zusätzliche äquimolare Menge an NaCl die Reibungsminderung von $C_{14}$TA-heptan-1-sulfonat nicht.

**Beispiel 8**

Wie in Beispiel 8 beschrieben wurden wässrige Lösungen von n-Alkyltrimethylammonium -n-alkyl-1-sulfonat mit unterschiedlichen Alkylresten durch Einwiegen der Salze n-Alkyltrimethylammoniumchlorid und Na-alkyl-1-sulfonat im molaren Mischungsverhältnis 1 : 1 hergestellt. In Tabelle 5 sind die Ergebnisse der Untersuchung, auf Reibungsminderung mit dem Turbulenzrheometer für die verschiedenen Tenside zusammengefaßt. Die äquimolaren Mengen an NaCl, die die Lösungen zusätzlich enthalten, wurden nicht mit aufgeführt.

**Beispiel 9**

Es wurden wässrige Lösungen von n-Hexadecyltrimethylammonium-3-methyl-salicylat (abgekürzt $C_{16}$TA-3-methyl-Sal), wie in Beispiel 5 beschrieben, angesetzt und vorbehandelt und im Turbulenzrheometer bei verschiedenen Temperaturen auf Reibungsminderung untersucht. Tabelle 6 faßt die Ergebnisse zusammen. In Abb. 1 sind die Fließkurven bei 50°C für 150, 300 und 1000 ppm Lösungen von $C_{16}$TA-3-methyl-Sal dargestellt. (1) zeigt die Meßkurve für reines Wasser.

**Beispiel 10**

Es wurden wässrige Lösungen von n-Hexadecyltrimethylammonium-4-methyl-salicylat (abgekürzt $C_{16}$TA-4-methyl-Sal), wie in Beispiel 5 beschrieben, angesetzt und vorbehandelt und im Turbulenzrheometer bei verschiedenen Temperaturen auf Reibungsminderung untersucht. Wie aus der Zusammenfassung der Ergebnisse in Tabelle 7 zu entnehmen ist, wurde Reibungsminderung bei 24°C bereits ab 60 ppm gefunden.

**Beispiel 11**

Es wurden wässrige Lösungen von n-Hexadecyltrimethylammonium-4-ethoxi-salicylat (abgekürzt $C_{16}$TA-4-ethoxi-Sal), wie in Beispiel 5 beschrieben, angesetzt und im Turbulenzrheometer auf Reibungsminderung untersucht. Tabelle 8 faßt die Ergebnisse zusammen.

**Beispiel 12**

Es wurden wässrige Lösungen von n-Hexadecyltrimethylammonium-3-oxy-4-methyl-benzoat (abgekürzt $C_{16}$TA-3-oxy-4-methyl-benzoat), wie im Beispiel 5 beschrieben, angesetzt und im Turbulenzrheometer bei 23°C auf Reibungsminderung untersucht. Tabelle 9 faßt die Ergebnisse zusammen.

**Beispiel 13**

Es wurden wässrige Lösungen von n-Hexadecyltrimethylammonium-2-hydroxy-1-naphthoat ($C_{16}$TA-2-hydroxy-1-naphthoat), wie in Beispiel 5 beschrieben, angesetzt und im Turbulenzrheometer bei 68°C auf Reibungsminderung untersucht. Tabelle 10 faßt die Ergebnisse zusammen.

**Beispiel 14**

Es wurden wässrige Lösungen von n-Hexadecyltrimethylammonium-3-hydroxy-2-naphthoat ($C_{16}$TA-3-hydroxy-2-naphthoat), wie in Beispiel 5 beschrieben, angesetzt und vorbehandelt und im Turbulenzrheometer bei 68, 80 und 93°C auf Reibungsminderung untersucht. wie auf Tabelle 11 ersichtlich, die die Meßergebnisse zusammenfaßt, zeigt dieses Tensid auch oberhalb von 90°C gute Wirksamkeit als SB.

**Beispiel 15**

Unterschiedliche Mengen an $Na_2CO_3$ wurden zusammen mit $C_{16}$TA-3-hydroxy-2-naphthoat, wie in Beispiel 6 beschrieben, angesetzt. Die Konzentration an $C_{16}$TA-3-hydroxy-2-naphthoat betrug jeweils 200 ppm (4,25.10 mol/l) und die Konzentrationen an $Na_2CO_2$ (in mol/l) :

$1.10^{-4}$ ; $2.10^{-4}$ ; $4{,}24.10^{-4}$ ; 0,005 ; 0,05 ; 0,5.

Zwei weitere 200 ppm Lösungen von $C_{16}$TA-3-hydroxy-2-naph-thoat wurden mit NaOH auf die pH-Werte 10,6 und 11,4 eingestellt und dann ebenfalls, wie in Beispiel 5 beschrieben, vorbehandelt. Alle Lösungen wurden bei 65°C im Turbulenzrheometer auf Reibungsminderung untersucht. Tabelle 12 faßt die Ergebnisse zusammen.

**Beispiel 16**

Wie in Beispiel 7 beschrieben, wurden wässrige Lösungen von n-Alkyltrimethylammonium-3-hydroxy-2-naphthoat ($C_n$TA-3-hydroxy-2-naphthoat) mit unterschiedlichen Alkylresten durch Einwiegen der Salze n-Alkyltrimethylammoniumchlorid, 3-Hydroxy-2-naphthoesäure und NaOH im molaren Verhältnis 1 :1 :1 hergestellt und im Turbulenzrheometer auf Reibungsminderung untersucht. Tabelle 13 faßt die Ergebnisse zusammen. Die äquimolaren Mengen an NaCl, die die Lösungen zusätzlich enthalten, wurden nicht gesondert aufgeführt.

**Beispiel 17**

Wie in Beispiel 16 und 7 beschrieben, wurden wässrige Lösungen verschiedener Tenside, wie $C_{14}$TA-naphthalin-1-hydroxy-2-sulfonat, $C_{14}$Ta-3-chlor-benzoat, $C_{16}$TA-4-methyl-benzoat, $C_{16}$TA-3-nitro-benzoat und $C_{16}$TA-naphthalin-1-hydroxy-2-sulfonat, durch Einwiegen der jeweiligen Salze n-Alkyltrimethylammoniumchlorid, der aromatischen Säuren und NaOH im molaren Verhältnis 1 :1 :1 hergestellt und im Turbulenzrheometer auf Reibungsminderung untersucht. Tabelle 14 faßt die Ergebnisse zusammen. Die äquimolaren Mengen an NaCl, die die Lösungen enthalten, wurden nicht mit aufgeführt.

**Beispiel 18**

Zur Untersuchung der Reibungsminderung in einem Dauerversuch bei 84 bis 90°C wurde eine Strömungsapparatur benutzt, bestehend aus einem 400 l Vorratsgefäß, einer Kreiselpumpe (Typ : CPK 50 - 250 der Firma KSB, Umdrehungszahl des Rotors 1450 U/min), einem induktiven Durchflußmesser und einer Rohrleitung von 20 m Länge mit einem Innendurchmesser von 29,75 mm. Die Bestimmung des Druckabfalls $\Delta P$ erfolgt über eine Meßstrecke von 1 m. Zur Thermostatisierung dient ein Tauchsieder, der die Flüssigkeit im Vorratsbehälter elektrisch aufheizt. Während des Dauerversuches wird die Flüssigkeit ständig am Boden des Vorratsgefäßes abgepumpt und über die Rohrleitung dem Vorratsbehälter wieder zugeführt. Für E-Wasser bei 84°C beträgt die Förderleistung der Pumpe 14,7 $m^3/h$, entsprechend einer Strömungsgeschwindigkeit u = 5,88 m/s, und der Druckabfall $\Delta P$ = 7000 Pa.

Die Prüfung der Zeitbeständigkeit des Strömungsbeschleunigers n-Hexa-decyltrimethylammonium-3-hydroxy-2-naphthoat in wässrige Lösung bei einer Konzentration von 640 ppm erfolgte in dieser Apparatur bei 84 bis 90°C. Hierzu wurde in die laufende Apparatur zu 400 l E-Wasser von 84°C 198 g Hexadecyltrimethylammonium-bromid, 102 g 3-Hydroxy-2-naphthoesäuse und 21,7 g NaOH, d.h. alle drei Salze im molaren Mischungsverhältnis 1 :1 :1, zugegeben. Durch Einwiegen einer zusätzlichen äquimolaren Menge an $Na_2CO_3$ (57,6 g) erfolgte eine Einstellung des pH-Wertes auf 10. Durch das ständige Umpumpen der Lösung stellte sich bereits 10 Minuten nach Zugabe der Salze die volle Wirkung als Strömungsbeschleuniger ein, die sich darin äußerte, daß die Förderleistung der Pumpe auf 21 $m^3/h$, entsprechend einer Strömungsgeschwindigkeit von 8,5 m/s, stieg und zusätzlich der Druckabfall über die Meßstrecke auf $\Delta P = 4200$ Pa abnahm.

Die % Reibungsminderung $\alpha$ wurde nach den bereits aufgeführten Gleichungen (Seite 14) berechnet, wobei allerdings der Reibungsbeiwert für reines E-Wasser, $\lambda H_2O$, theoretisch für die Re-Zahl berechnet wurde, die sich aus der gestiegenen Förderleistung der Pumpe nach der Zugabe des SB ergab.

Tabelle 15 faßt die Ergebnisse des Dauerversuches, die Zeit in Tagen, die Temperatur T, die Reynoldszahl Re und die % Reibungsminderung $\alpha$ zusammen.

Wie die Ergebnisse zeigen ist über einen Zeitraum von 14 Tagen bein einer Temperatur von 84 - 90°C selbst bei Verwendung einer Kreiselpumpe, die den Inhalt des Vorratsgefäßes pro Stunde 53,5 mal bzw. pro Tag 1284 mal umpumpt, keine Abnahme in der SB-Wirkung festzustellen.

**Beispiel 19**

Für Messungen über 100°C wurden wie in Beispiel 7 beschrieben wässrige Lösungen von n-Alkyltrimethylammonium-3-hydroxy-2-naphthoat ($C_nTA$-3-hydroxy-2-naphthoat) durch Einwiegen der Salze n-Alkyltrimethylammoniumchlorid, 3-Hydroxy-2-naphthoesäure und NaOH im molaren Verhältnis 1 :1 :1 hergestellt. Im Unterschied zu Beispiel 5 wurden diese Lösungen jedoch nur für ca. 20 Stunden bei 95°C gelagert und anschließend vor jeder Messung im Turbulenzrheometer jeweils eine halbe Stunde bei der jeweiligen Meßtemperatur thermostatisiert. Wie aus der Zusammenfassung der Ergebnisse der Untersuchung auf Reibungsminderung im Turbulenzrheometer in Tabelle 16 zu entnehmen ist, wurde Reibungsminderung im Temperaturbereich von 110 bis 130°C gefunden. Auch nach wiederholtem Aufheizen und Messen der Lösungen bei den jeweiligen Temperaturen blieb die Wirkung als SB erhalten.

**Beispiel 20**

Es wurde eine Konzentrationsreihe von 500, 750 und 1500 Gew.-ppm von Hexadecyltrimethylammonium-2-phenol-sulfonat ($C_{16}TA$-2-phenolsulfonat), wie in Beispiel 5 beschrieben, angesetzt und vorbehandelt und im Turbulenzrheometer bei 50°C gemessen. Eine Lösung mit einer Konzentration von 3000 Gew.-ppm an $C_{16}TA$-2-phenolsulfonat in Wasser wurde bei 65°C gemessen. Tabelle 17 faßt die Ergebnisse zusammen.

**Beispiel 21**

Unterschiedliche Mengen an NaCl wurden zusammen mit $C_{16}TA$-2-phenolfulfonat, wie in Beispiel 6 beschrieben, angesetzt und im Turbulenzrheometer bei 55°C auf Reibungsminderung untersucht. Die Konzentrationen an $C_{16}TA$-2-phenolsulfonat betrug jeweils 750 ppm (= $2{,}27.10^{-3}$mol/l) und die Konzentrationen an NaCl (in mol/l) : $5.10^{-5}$ ; $1.10^{-4}$ ; $5.10^{-4}$ ; $2{,}27.10^{-3}$ ; $6.10^{-3}$ ; $1.10^{-2}$. Tabelle 18 faßt die Ergebnisse zusammen.

Tabelle 1

$C_{14}$TA-heptan-1-sulfonat

| T [°C] | Konzentration [ppm] | $Re_{max}$ | $\alpha_{max}$ (% Reibungsminderung) |
|---|---|---|---|
| 23 | 300 | 6100 ± 600 | 60 ± 3 |
| 23 | 500 | 9800 ± 1000 | 65 ± 3 |
| 23 | 750 | 15100 ± 1500 | 68 ± 3 |
| 23 | 1000 | 19200 ± 1900 | 68 ± 3 |
| 23 | 1500 | 22700 ± 2300 | 71 ± 4 |

Tabelle 2

$C_{14}$TA-heptan-1-sulfonat

| T [°C] | Konzentration [ppm] | $Re_{max}$ | $\alpha_{max}$ (% Reibungsminderung) |
|---|---|---|---|
| 35 | 300 | - | kein Effekt |
| 35 | 500 | - | kein Effekt |
| 35 | 750 | 12100 ± 1200 | 21 ± 1 |
| 35 | 1000 | 17000 ± 1700 | 42 ± 2 |
| 35 | 1500 | 19900 ± 2000 | 46 ± 2 |

Tabelle 3

Meßtemperatur 23°C, $C_{14}$TA-heptan-1-sulfonat-Konz.: 750 ppm

| $Na_2CO_3$-Konz. [mol/l] | $Re_{max}$ | $\alpha_{max}$ |
|---|---|---|
| $1 \cdot 10^{-4}$ | 14100 ± 1400 | 67 ± 3 |
| $2 \cdot 10^{-4}$ | 15400 ± 1500 | 68 ± 3 |
| $1 \cdot 10^{-3}$ | 14700 ± 1500 | 68 ± 3 |
| $1,72 \cdot 10^{-3}$ | 15800 ± 1600 | 68 ± 3 |
| 0,01 | 12200 ± 200 | 67 ± 3 |
| 0,1 | 6500 bis 19000 | 20 bis 30 |

Tabelle 4

Meßtemperatur 23°C, 1 : 1 molare Mischungen aus $C_{14}$TACl und Na-heptan-1-sulfonat.

| Konzentration der Mischung [ppm] | $Re_{max}$ | $\alpha_{max}$ |
|---|---|---|
| 500 | 9900 ± 1400 | 67 ± 3 |
| 1000 | 16600 ± 1700 | 68 ± 3 |
| 1500 | 20800 ± 2100 | 68 ± 3 |
| 2000 | 23500 ± 2400 | 70 ± 4 |

Tabelle 5

| Tensid | Konzentration [ppm] | Temperatur [°C] | $Re_{max}$ | $\alpha_{max}$ |
|---|---|---|---|---|
| $C_{16}TA^{\oplus}$ $C_6H_{13}SO_3^{\ominus}$ | 2000 | 22 | 7900 ± 800 | 56 ± 3 |
| $C_{18}TA^{\oplus}$ $C_6H_{13}SO_3^{\ominus}$ | 2000 | 22 | 4500 ± 500 | 24 ± 1 |
| $C_{22/22}TA^{\oplus}$ $C_6H_{13}SO_3^{\ominus}$ | 2000 | 45 | 33600 ± 3400 | 70 ± 4 |
| $C_{16}TA^{\oplus}$ $C_7H_{15}SO_3^{\ominus}$ | 1000 | 45 | 13600 ± 1400 | 56 ± 3 |
| $C_{18}TA^{\oplus}$ $C_7H_{15}SO_3^{\ominus}$ | 1000 | 45 | 6700 ± 700 | 47 ± 2 |
| $C_{20/22}TA^{\oplus}$ $C_7H_{15}SO_3^{\ominus}$ | 1000 | 80 | 4000 ± 400 | 35 ± 2 |
| $C_{14}TA^{\oplus}$ $C_8H_{17}SO_3^{\ominus}$ | 2000 | 45 | 18200 ± 1800 | 70 ± 4 |
| $C_{16}TA^{\oplus}$ $C_8H_{17}SO_3^{\ominus}$ | 2000 | 45 | 41800 ± 4200 | 75 ± 4 |
| $C_{18}TA^{\oplus}$ $C_8H_{17}SO_3^{\ominus}$ | 2000 | 80 | 46000 ± 4600 | 73 ± 4 |
| $C_{20/22}TA^{\oplus}$ $C_8H_{15}SO_3^{\ominus}$ | 2000 | 90 | 17800 ± 1800 | 68 ± 3 |

$C_nTA^+$ steht jeweils für $[C_nH_{2n+1}N(CH_3)_3]^{\oplus}$

Tabelle 6

$C_{16}$TA-3-methyl-Sal

| T [°C] | Konzentration [ppm] | $Re_{max}$ | $\alpha_{max}$ |
|---|---|---|---|
| 23 | 100 | 6700 ± 700 | 59 ± 3 |
| 23 | 150 | 8700 ± 900 | 61 ± 3 |
| 23 | 300 | 8900 ± 900 | 63 ± 3 |
| 23 | 1500 | 12100 ± 1200 | 63 ± 3 |
| 50 | 150 | 9800 ± 1000 | 62 ± 3 |
| 50 | 300 | 16700 ± 1700 | 66 ± 3 |
| 50 | 1000 | 33800 ± 3400 | 74 ± 4 |
| 68 | 300 | 17900 ± 1800 | 65 ± 3 |
| 68 | 1000 | 34100 ± 3400 | 71 ± 4 |

Tabelle 7

$C_{16}$TA-4-methyl-Sal

| T [°C] | Konzentration [ppm] | $Re_{max}$ | $\alpha_{max}$ |
|---|---|---|---|
| 24 | 60 | 4400 ± 400 | 57 ± 3 |
| 24 | 80 | 6300 ± 600 | 56 ± 3 |
| 24 | 90 | 6900 ± 700 | 57 ± 3 |
| 22 | 100 | 6300 ± 600 | 58 ± 3 |
| 22 | 150 | 9000 ± 900 | 64 ± 3 |
| 22 | 300 | 10800 ± 1100 | 63 ± 3 |
| 22 | 1000 | 10900 ± 1100 | 64 ± 3 |
| 50 | 150 | 10100 ± 1000 | 61 ± 3 |
| 50 | 300 | 17100 ± 1700 | 67 ± 3 |
| 50 | 1000 | 34400 ± 3400 | 74 ± 4 |
| 68 | 300 | 15500 ± 1500 | 65 ± 3 |
| 68 | 1000 | 34200 ± 3400 | 72 ± 4 |

Tabelle 8

$C_{16}$TA-4-ethoxi-Sal

| T [°C] | Konzentration [ppm] | $Re_{max}$ | $\alpha_{max}$ |
|---|---|---|---|
| 24 | 100 | 6000 ± 600 | 59 ± 3 |
| 24 | 150 | 9000 ± 900 | 58 ± 3 |
| 24 | 300 | 13200 ± 1300 | 68 ± 3 |
| 24 | 500 | 15700 ± 1600 | 66 ± 3 |
| 45 | 2000 | 45900 ± 4600 | 77 ± 4 |

Tabelle 9

$C_{16}$TA-3-oxy-4-methyl-benzoat

| T [°C] | Konzentration [ppm] | $Re_{max}$ | $\alpha_{max}$ |
|---|---|---|---|
| 23 | 100 | 5800 ± 600 | 62 ± 3 |
| 23 | 150 | 6700 ± 700 | 60 ± 3 |
| 23 | 300 | 9400 ± 900 | 65 ± 3 |
| 23 | 1000 | 11700 ± 1200 | 65 ± 3 |
| 23 | 2000 | 17700 ± 1800 | 69 ± 4 |

Tabelle 10

$C_{16}$TA-2-hydroxy-1-naphthoat

| T [°C] | Konzentration [ppm] | $Re_{max}$ | $\alpha_{max}$ |
|---|---|---|---|
| 68 | 200 | 16000 ± 1600 | 67 ± 3 |
| 68 | 300 | 22100 ± 2200 | 70 ± 4 |
| 68 | 400 | 32800 ± 3300 | 72 ± 4 |
| 68 | 750 | 43400 ± 4300 | 72 ± 4 |
| 68 | 1000 | 60400 ± 6000 | 78 ± 4 |

Tabelle 11

$C_{16}$TA-3-hydroxy-2-naphthoat

| T [°C] | Konzentration [ppm] | $Re_{max}$ | $\alpha_{max}$ |
|---|---|---|---|
| 68 | 100 | 11000 ± 1200 | 59 ± 3 |
| 68 | 200 | 18800 ± 1900 | 68 ± 3 |
| 68 | 300 | 29700 ± 3000 | 72 ± 4 |
| 68 | 400 | 35300 ± 3500 | 75 ± 4 |
| 68 | 500 | 41700 ± 4200 | 75 ± 4 |
| 68 | 750 | 49800 ± 5000 | 77 ± 4 |
| 68 | 1000 | 59200 ± 6000 | 75 ± 4 |
| 80 | 200 | 14700 ± 1500 | 66 ± 3 |
| 80 | 300 | 21400 ± 2100 | 68 ± 3 |
| 80 | 400 | 29800 ± 3000 | 70 ± 4 |
| 80 | 500 | 35300 ± 3500 | 72 ± 4 |
| 80 | 750 | 48300 ± 4800 | 75 ± 4 |
| 80 | 1000 | 57000 ± 5700 | 75 ± 4 |
| 93 | 500 | 28000 ± 2800 | 77 ± 4 |
| 93 | 750 | 40000 ± 4000 | 79 ± 4 |
| 93 | 1000 | 52000 ± 5200 | 74 ± 4 |

Tabelle 12

Temperatur 65°C, $C_{16}$TA-3-hydroxy-2-naphthoat-Konz.: 200 ppm

| $Na_2CO_3$-Konz. [mol/l] | pH | $Re_{max}$ | $\alpha_{max}$ |
|---|---|---|---|
| $1 \cdot 10^{-4}$ | 7,6 | 18300 ± 1800 | 65 ± 3 |
| $2 \cdot 10^{-4}$ | 10,1 | 19200 ± 1900 | 67 ± 3 |
| $4,24 \cdot 10^{-4}$ | 10,3 | 20400 ± 2000 | 68 ± 3 |
| $5 \cdot 10^{-3}$ | 10,8 | 19700 ± 2000 | 70 ± 4 |
| 0,05 *) | 11,4 | 18100 ± 1800 | 68 ± 3 |
| 0,5 *) | 11,7 | 15100 ± 1500 | 63 ± 3 |
| NaOH-Zugabe | 10,6 | 20100 ± 2000 | 63 ± 3 |
| | 11,4 | 17300 ± 1700 | 65 ± 3 |

*) Lösungen enthielten geringe Mengen an Niederschlag.

Tabelle 13

$C_n$TA-3-hydroxy-2-naphthoat + NaCl

| $C_n$TA | T [°C] | Konz. [ppm] | $Re_{max}$ | $\alpha_{max}$ |
|---|---|---|---|---|
| $C_{12}$TA | 45 | 2000 | 23200 ± 2300 | 47 ± 2 |
| $C_{14}$TA | 55 | 2000 | 52800 ± 5300 | 78 ± 2 |
| $C_{18}$TA | 90 | 300 | 21400 ± 2100 | 68 ± 3 |

$C_n$TA steht jeweils für $[C_nH_{2n+1}N(CH_3)_3]^+$

Tabelle 14

| Tensid | Konz. [ppm] | T [°C] | $Re_{max}$ | $\alpha_{max}$ |
|---|---|---|---|---|
| $C_{14}$TA-3-chlor-benzoat | 2000 | 22 | 22700 ± 2300 | 71 ± 4 |
| $C_{14}$TA-naphthalin-1-hydroxy-2-sulfonat | 2000 | 22 | 23100 ± 2300 | 36 ± 2 |
| $C_{16}$TA-4-methyl-benzoat | 5000 | 22 | 25800 ± 2600 | 65 ± 3 |
| $C_{16}$TA-3-nitro-benzoat | 5000 | 22 | 8600 ± 900 | 50 ± 3 |
| $C_{16}$TA-naphthalin-1-hydroxy-2-sulfonat | 2000 | 22 | 20300 ± 2000 | 69 ± 3 |

$C_n$TA steht jeweils für $[C_nH_{2n+1}N(CH_3)_3]^+$

Tabelle 15

Dauerversuch mit $C_{16}$TA-3-hydroxy-2-naphthoat (640 ppm)

| Zeit [Tage] | Temperatur [°C] | $Re_{max}$ | $\alpha_{max}$ |
|---|---|---|---|
| 0,01 | 84 | 727000 | 72 ± 4 |
| 4 | 85 | 740000 | 73 ± 4 |
| 5 | 84 | 732000 | 73 ± 4 |
| 6 | 90 | 773000 | 71 ± 4 |
| 7 | 90 | 773000 | 71 ± 4 |
| 10 | 90 | 769000 | 70 ± 4 |
| 12 | 90 | 765000 | 70 ± 4 |
| 14 | 90 | 729000 | 68 ± 3 |

Tabelle 16

| Tensid | T [°C] | Konz. [ppm] | $Re_{max}$ | $\alpha_{max}$ |
|---|---|---|---|---|
| $C_{18}$TA-3-hydroxy-2-naphthoat | 110 | 1000 | 60600 ± 6100 | 70 ± 4 |
| " | 115 | 1000 | 42600 ± 4300 | 62 ± 3 |
| $C_{20/22}$TA-3-hydroxy-2-naphthoat | 120 | 814 | 50200 ± 5000 | 60 ± 3 |
| " | 132 | 1000 | 34000 ± 3400 | 45 ± 2 |

$C_n$TA steht jeweils für $[C_nH_{2n+1}N(CH_3)_3]^+$

Tabelle 18

$C_{16}$TA-2-phenolsulfonat

| T [°C] | Konzentration [ppm] | $Re_{max}$ | $\alpha_{max}$ |
|---|---|---|---|
| 50 | 500 | 6700 ± 700 | 58 ± 3 |
| 50 | 750 | 8500 ± 900 | 60 ± 3 |
| 50 | 1500 | 16400 ± 1600 | 65 ± 3 |
| 65 | 3000 | 36600 ± 3700 | 71 ± 4 |

Tabelle 19

Meßtemperatur 55°C, $C_{16}$TA-2-phenolsulfonat-Konz.: 750 ppm

| NaCl-Konz. [mol/l] | $Re_{max}$ | $\alpha_{max}$ |
|---|---|---|
| $5.10^{-5}$ | 7400 ± 700 | 65 ± 3 |
| $1.10^{-4}$ | 10100 ± 1000 | 64 ± 3 |
| $5.10^{-4}$ | 12200 ± 1200 | 67 ± 3 |
| $2.27\ 10^{-3}$ | 12100 ± 1200 | 71 ± 4 |
| $6.10^{-3}$ | 11600 ± 1200 | 44 ± 2 |
| 0,01 | 20200 ± 2000 | 70 ± 4 |
| 0,05 | keine Reibungsminderung | |

**Patentansprüche**

1. Quartäre Ammoniumsalze der Formel

$R_1\text{-}K^{\oplus}\ A^{\ominus}$

wobei $R_1$ $C_{12}$ - $C_{26}$-Alkyl oder $C_{12}$ - $C_{26}$-Alkenyl, $K^{\oplus}$ eine Gruppe der Formel

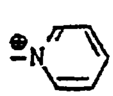

oder $-^{\oplus}N(R_2)_3$, $R_2$ $C_1$-$C_3$-Alkyl, vorzugsweise Methyl und $A^{\ominus}$ ein Anion der folgenden Formeln bedeutet: $R_3SO_3^{\ominus}$, wo $R_3$ $C_6$-$C_9$-Alkyl oder Alkenyl ist und die Summe der C-Atome in $R_1$ und $R_3$ mindestens 21 betragen

soll ;

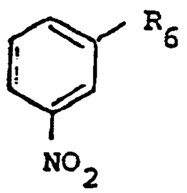 ; 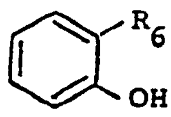 ; 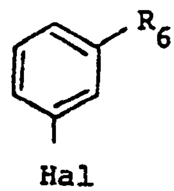 ; 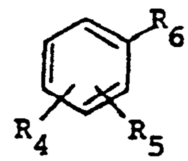 oder

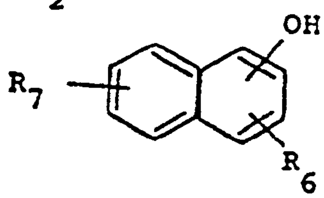

wo Hal Fluor Chlor, Brom oder Jod $R_4$ $C_1$ - $C_5$-Alkyl, $C_2$ - $C_5$-Alkenyl oder $C_1$ - $C_5$-Alkoxy in den Stellungen 3, 4, 5 und 6, $R_5$ Wasserstoff oder Hydroxy in den Stellungen 2 und 3, $R_6$ $COO^{\ominus}$ oder für den Fall $R_5$ = OH auch $SO_3^{\ominus}$ und $R_7$ Wasserstoff oder Methyl ist, mit der Maßgabe, daß $A^{\ominus}$ nicht ein Salicylat- oder m-Halogenbenzoat-Ion ist, wenn $R_1$-$K^{\oplus}$ $C_{16}$-Alkyl-pyridinium, $C_{16}$-Alkenyl-pyridinium, $C_{14}$ - $C_{24}$-Alkyl-tri-$C_1$-$C_3$ alkylammonium und $C_{16}$-Alkenyltri-$C_1$-$C_3$-alkylammonium bedeutet.

2. Verfahren zur Herstellung der quartären Ammoniumsalze nach Anspruch 1, dadurch gekennzeichnet, daß man eine Verbindung der Formel

$$R_1\text{-}K^{\oplus}\ Hal^{\ominus}$$

wobei $Hal^{\ominus}$ ein Chlor-, Brom- oder Jod-Anion bedeutet, mit Silberhydroxid umsetzt, das ausgefallene Silberhalogenid abtrennt und die erhaltene Verbindung der Formel

$$R_1\text{-}K^{\oplus}\ OH^{\ominus}$$

mit einer das Anion $A^-$ enthaltenden Säure neutralisiert.

3. Verwendung der quartären Ammoniumsalze nach Anspruch 1 als Strömungsbeschleuniger in turbulen strömenden wäßrigen Medien.

## Claims

1. A quaternary ammonium salt of the formula

$$R_1\text{-}K^{\oplus}A^{\ominus}$$

in which $R_1$ denotes $C_{12}$-$C_{26}$-alkyl or $C_{12}$-$C_{26}$-alkenyl, $K^{\oplus}$ denotes a group of the formula

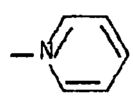

or $-^{\oplus}N(R_2)_3$, $R_2$ denotes $C_1$-$C_3$-alkyl, preferably methyl, and $A^{\ominus}$ denotes an anion of one of the following formulae :

$R_3SO_3^{\ominus}$, in which $R_3$ is $C_8$-$C_9$-alkyl or alkenyl and the sum of the C atoms in $R_1$ and $R_3$ should be at least 21 ;

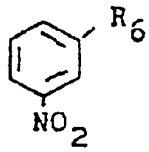 ; 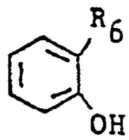 ; 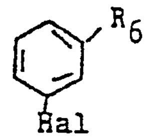 ; 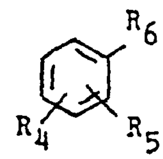

or 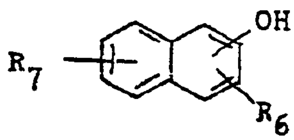

in which Hal is fluorine, chlorine, bromine or iodine, $R_4$ is $C_1$-$C_5$-alkyl, $C_2$-$C_5$-alkenyl or $C_1$-$C_5$-alkoxy in positions 3, 4, 5 or 6, $R_5$ is hydrogen or hydroxyl in positions 2 or 3, $R_5$ is $COO^\ominus$ or, if $R_5$ is hydroxy, $R_6$ is also $SO_3^\ominus$ and $R_7$ is hydrogen or methyl, with the proviso that $A^\ominus$ is not a salicylate or m-halogenobenzoate ion when $R_1$-$K^\oplus$ is $C_{16}$-alkyl-pyridinium, $C_{16}$-alkenyl-pyridinium, $C_{14}$-$C_{24}$-alkyl-tri-$C_1$-$C_3$-alkylammonium and $C_{16}$-alkenyltri-$C_1$-$C_3$-alkylammonium.

2. A process for the preparation of a quaternary ammonium salt as claimed in claim 1, which comprises reacting a compound of the formula

$$R_1\text{-}K^\oplus Hal^\ominus$$

in which $Hal^\ominus$ denotes a chlorine, bromine or iodine anion, with silver hydroxide, separating off the precipitated silver halide and neutralizing the resulting compound of the formula

$$R_1\text{-}K^\oplus\ OH^\ominus$$

with an acid containing the anion $A^\ominus$.

3. The use of a quaternary ammonium salt as claimed in claim 1 as a drag reducing agent in an aqueous medium with turbulent flow.

## Revendications

1. Sels d'ammoniums quaternaires répondant à la formule :

$$R_1\text{-}K^\oplus\ A^\ominus$$

dans laquelle $R_1$ représente un alkyle en $C_{12}$-$C_{26}$ ou un alcényle en $C_{12}$-$C_{26}$, $K^\oplus$ représente un radical de formule

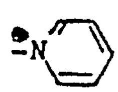

ou $-^\oplus N(R_2)_3$, où $R_2$ représente un alkyle en $C_1$-$C_3$, de préférence un méthyle, et $A^\ominus$ représente un anion répondant à l'une des formules suivantes :

$R_3SO_3^\ominus$, où $R_3$ représente un alcényle ou un alkyle en $C_8$-$C_9$, la somme des atomes de carbone contenus dans $R_1$ et $R_3$ devant au moins être égale à 21,

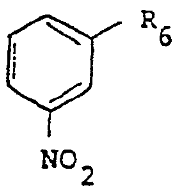 ; 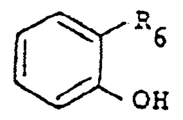 ; 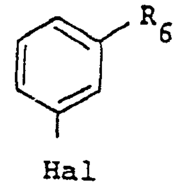 ; 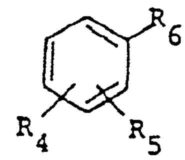 et

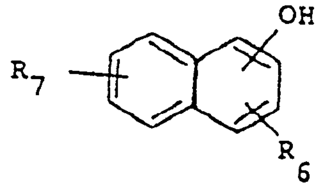

où Hal représente le fluor, le chlore, le brome ou l'iode, $R_4$ représente un alkyle en $C_1$-$C_5$, un alcényle en $C_2$-$C_5$ ou un alcoxy en $C_1$-$C_5$ et peut se trouver en l'une des positions 3, 4, 5 et 6, $R_5$ représente l'hydrogène ou un radical hydroxy qui se trouve à la position 2 ou à la position 3, $R_6$ représente un radical -COO$^\ominus$ et, dans le cas où $R_5$ représente un radical hydroxy peut également représenter -$SO_3^\ominus$, et $R_7$ représente l'hydrogène ou un méthyle, avec la condition que $A^\ominus$ ne soit pas un ion salicylate ou m-halogéno-benzoate lorsque $R_1$-$K^\oplus$ représente un radical $C_{16}$-alkyl-pyridinium, $C_{16}$-alcényl-pyridinium, $C_{14}$-$C_{24}$-alkyl-tris-($C_1$-$C_3$-alkyl)-ammonium ou $C_{16}$-alcényl-tris-($C_1$-$C_3$-alkyl)-ammonium.

2. Procédé de préparation des sels d'ammoniums quaternaires selon la revendication 1, procédé caractérisé en ce qu'on fait réagir un composé répondant à la formule :

$$R_1\text{-}K^\oplus\ Hal^\ominus$$

dans laquelle $Hal^\ominus$ représente un anion chlore, brome ou iode, avec l'hydroxyde d'argent, on sépare l'halogénure d'argent qui a précipité et on neutralise le composé obtenu, qui répond à la formule suivante :

$$R_1\text{-}K^\oplus\ OH^\ominus,$$

avec un acide contenant l'anion $A^\ominus$.

3. Application des sels d'ammoniums quaternaires selon la revendication 1 comme accélérateurs d'écoulement dans des milieux liquides en écoulement turbulent.

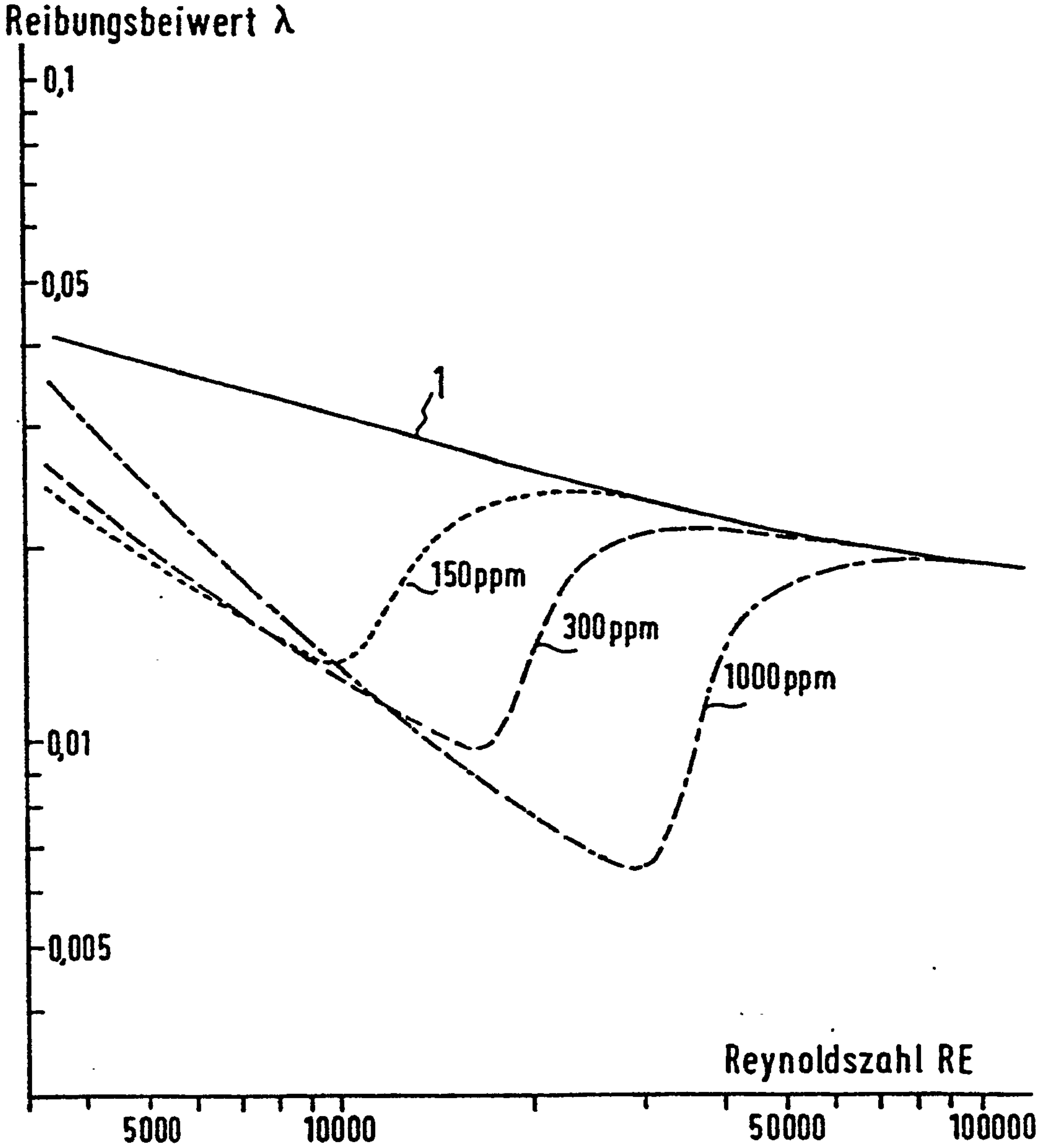
Reibungsbeiwert λ
0,1
0,05
0,01
0,005
1
150ppm
300ppm
1000ppm
Reynoldszahl RE
5000
10000
50000
100000